# EUROPEAN PATENT APPLICATION

(11) **EP 2 322 141 A2**
(43) Date of publication of application: **18.05.2011**
(21) Application number: 10186651.5
(22) Date of filing: 06.10.2010
(51) Int. Cl.: A61K 8/91, A61Q 5/06, C08J 3/24

(54) **Cosmetic compositions based on elastomeric polymers**

(30) Priority: 07.10.2009 EP 09172426
(71) Applicant: The Procter & Gamble Company, Cincinnati, OH 45202 (US)
(72) Inventor: Flohr, Andreas, 61476, Kronberg im Taunus (DE); Kripp, Thomas, 64407, Fränkisch-Crumbach (DE); Morand, Matthias, 65812, Bad Soden (DE)
(74) Representative: Marollé, Patrick Pierre Pascal

(57) **Abstract**

Cosmetic compositions comprising elastomeric polymers comprising chain segments being bound to each other via direct covalent bonds, and a cosmetically acceptable carrier. Use of said elastomeric polymers for providing elastic properties to a cosmetic composition. Use of said cosmetic compositions for providing long-lasting hold, high-setting properties, and elasticity to the hair style. Methods for preparing said cosmetic composition and for applying said cosmetic composition onto hair and/or skin.

## Description

### FIELD OF THE INVENTION

According to a first aspect, the present invention relates to a cosmetic composition comprising elastomeric polymers comprising chain segments being bound to each other via direct covalent bonds, and a cosmetically acceptable carrier. According to a second aspect, the present invention also relates to the use of said elastomeric polymers for providing elastic properties to a cosmetic composition. According to a third aspect, the present invention relates to cosmetic compositions for providing long-lasting hold, high-setting properties, and elasticity to the hair style. According to further aspects, the present invention relates to methods for preparing said cosmetic composition comprising said elastomeric polymers comprising chain segments being bound to each other via direct covalent bonds, and a cosmetically acceptable carrier, as well as a method for applying said cosmetic composition onto skin and/or hair. The cosmetic composition, according to the present invention, may be used as a skin care composition, a shave care composition and/or a hair care composition.

### BACKGROUND OF THE INVENTION

Cosmetic compositions are commonly used for beautifying and/or treating the skin and/or keratinous materials, e.g. hair, of the users. Depending on the benefit expected, various types of compositions are available on the market, e.g. skin and/or hair care compositions, as well as skin cleansing, hair cleansing, hair styling, hair conditioning, hair dyeing and/or shaving compositions. Again, depending on the benefit expected, these compositions may be of various forms, e.g. liquid or solid compositions. They may also comprise various types of components.

One of the most common types of components is polymer. Polymers comprise a backbone - or chain - of polymerized units, said units being derived from monomers. Polymers may be obtained by polymerization of one type of monomers, i.e. homopolymers, or at least two different types of monomers, i.e. copolymers. Common monomers are acrylic acid monomer, acrylamide monomer, vinyl pyrrolidone monomer, styrene monomer, their salts, their derivatives, or mixtures thereof. Depending on the class of monomers, their specific structure, their degree of insaturation, their degree of neutralisation, their proportions in the polymer, and/or the polymerization process, polymers exhibit specific properties and provide specific benefits. In order to customize further polymer properties and/or in order to render polymers more suitable for targeted applications, these polymers may be modified further, e.g. by grafting chemical groups and/or by cross-linking the polymeric backbone using cross-linking agents.

It is known various polymers, having various properties and exhibiting various benefits, suitable for incorporation into cosmetic compositions. Extensive information about marketed polymers is listed in the International Cosmetic Ingredient Dictionary and Handbook, 12th edition (2008) from the Personal Care Products Council (formerly the Cosmetic, Toiletry and Fragrance Association or CTFA). For example, polymers may be emulsion stabilizers, suspending agents, viscosity increasing agents, opacifying agents, film-forming agents, hair-fixative agents, skin moisturizing agents and/or hair conditioning agents.

A conventional cross-linking process usually consists of forming a cross-link between two cross-linkable units using a cross-linking agent. When two units comprised within two different polymeric backbones are cross-linked to each other, an inter-chain cross-link is obtained. When two units comprised within one polymeric backbone are cross-linked to each other, an intra-chain cross-link is obtained. Cross-linking agents are at least bi-functional molecules which form, usually upon activation, two covalent bonds with two different units of the polymerized backbone. The cross-linking agent itself gets incorporated into the final cross-linked polymer and constitutes the cross-link. These cross-links are designated herewith as "indirect cross-links". As a result, these "indirect cross-links" comprise intermediary molecules - said molecules being of various chain lengths - coming from the cross-linking agents themselves.

Concerning the synthesis of these cross-links, there are two common ways to obtain cross-linked polymers.

A first way is an in-situ-cross-linking during the polymerisation reaction. This can be done easily by adding to a certain percentage of cross-linking agents - being "dimers" - into the polymerisation batch. A conventional cross-linking agent for polymers suitable for cosmetic applications is methylene-bis-acrylamide, of formula (CH₂=CHCONH)₂CH₂. Methylene-bis-acrylamide is particularly suitable for forming indirect cross-links, between two units derived from acrylamide monomers. Other conventional cross-linking agents are, for example, di(meth)acrylate, N-(1-hydroxy-2,2-dimethoxyethyl)acrylamide, ethyleneglycol dimethacrylate, ethyleneglycol diacrylate, allylmethacrylate, 1,1,1-trimethylolpropane triacrylate, triallylamine, and tetraallyloxethane.

The second way is a cross-linking carried out after treatment via derivatisation of reactive groups of the polymer. For example, in case of the presence of acidic groups as present in poly(acrylic acid), it is possible to form diester-bridges by means of diol cross-linking agents or diamide-bridges by the help of diamine cross-linking agents.

Both methods lead to cross-linked polymers with a more or less extensive space between the single polymer chains. Bridges which are based on ester, ether or amide bonds are usually prone to cleavage by hydrolysis which might lead to undesired drawbacks in terms of stability, especially in aqueous media.

Cross-linking polymeric backbones using cross-linking agents modifies polymer properties, e.g. its water-solubility, molecular weight, glass transition temperature, consistency, melting behavior and/or porosity. In addition, such cross-linking process usually alters elastic properties of polymers. In general, higher cross-linking density increases elasticity of polymers. However, a too high cross-linking density may render the cross-linked polymer brittle. The provision of polymers exhibiting increased rigidity and/or reduced elasticity is however not preferred for incorporation into cosmetic compositions as it impacts on the composition properties, its ease of application onto skin and/or hair and on the benefits conferred to skin and/or hair. Instead, it is preferred to use elastomeric polymers, i.e. polymers having satisfactory elastic properties. There is a need therefore for improving the elastic properties of commercially available polymers.

Particularly, hair styling compositions - used in order to achieve a hairstyle - are expected to provide satisfactory hold and setting properties to the hairstyle as well as providing sufficient elasticity to said hairstyle. However, hair styling compositions comprising currently available marketed hair styling polymers confer long-lasting hold and high-setting properties to the hairstyle, but a limited elasticity, if not at all. There is a need therefore for improving the elastic properties of commercially available hair styling polymers.

Alternative cross-linking processes are already known in different technical fields, e.g. processes for making superabsorbant polymers comprising polymer chain segments, which are directly bound to each other through covalent bonds. These superabsorbant materials are commonly applied in absorbent articles, such as diapers, training pants, adult incontinence products and feminine care products to increase the absorbent capacity of such products while reducing their overall bulk. See e.g. EP 1 568 385 A1.

There is a constant need for providing cosmetic compositions having an improved elasticity. Particularly, there is a need for providing cosmetic compositions having improved water-solubility, consistency and/or porosity as well as improved elastic properties. There is also a need for providing cosmetic compositions comprising cross-linked polymers being obtained via an alternative process to conventional cross-linking using cross-linking agents. Specifically, there is a need for providing hair styling compositions conferring long-lasting, high setting properties as well as elasticity to the hair style.

### BRIEF DESCRIPTION OF THE DRAWINGS

While the specification concludes with claims which particularly point out and distinctly claim the present invention, it is believed that the present invention will be better understood from the following description of preferred embodiments, taken in conjunction with the accompanying drawings, in which like reference numerals identify identical elements and wherein:
FIG. 1 is a graph showing the Loss modulus, the Storage modulus and the tan δ (loss modulus / storage modulus) function of the UV time (min) using DMA Test on poly(vinyl alcohol).
FIG. 2 is a graph showing the behaviour of tan δ (= loss modulus / storage modulus) using UV-Rheology measurements on Poly(vinylpyrrolidone).

### SUMMARY OF THE INVENTION

According to a first aspect, the present invention relates to a cosmetic composition comprising, in a cosmetically acceptable carrier, elastomeric polymers comprising from 1% to 100% of polymerized units (A),
wherein said units (A), in their non-cross linked form, comprise at least one carbon having an alpha-hydrogen relative to R₃ and have the general formula I 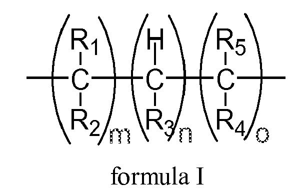 wherein R₃ is selected from the group consisting of:
   (a) a carboxyl derivative, a thiocarboxyl derivative;
   (b) a carbonyl derivative, a thiocarbonyl derivative, an imine derivative;
   (c) a hydroxyl derivative
   (d) an aryl radical, a heteroaryl radical;
   (e) a saturated cyclic radical;
   (f) an alkoxy derivative;
   (g) an amino derivative, an amide derivative, an imide derivative;
   (h) a phosphorus derivative;
   (i) a sulphur derivative;
   (j) a tin derivative;
   (k) a silica derivative;
   (l) a alkyne derivative;
   (m)a heterocyclic also incorporating R2;
wherein R₁, R₂, R₄, and R₅ are the same or different and are selected from the groups consisting of hydrogen, C₁-C₂₂ saturated alkyl derivatives, C₁-C₂₂ unsaturated alkyl derivatives, hydroxyl derivatives, C1-C₂₂ alkoxy derivatives, carboxyl derivatives, thiocarboxyl derivatives, amino derivatives, aminoalkyl derivatives, aminodialkyl derivatives, aminotrialkyl derivatives, tetraalkylammonium derivatives, amide derivatives, an imide derivatives, sulfide derivatives, sulfoxide derivatives, sulfone derivatives, phosphite derivatives, phosphone derivatives, phosphonate derivatives, tin derivatives and silica derivatives, aryl derivatives, C₁-C₇ aryl derivatives, heteroaryl derivatives, C₁-C₇ heteroaryl derivatives, cyano derivatives, alkynyl derivatives;
wherein indicia m, o, n are independent to each other with m ≥ 0, o ≥ 0 and n ≥ 1; and,
wherein direct covalent bonds are formed between the alpha carbons of two different units (A) by break of the alpha-hydrogen-bond relative to R₃ and wherein said bonds comprise no intermediate molecule.

According to a second aspect, the present invention relates to the use of the elastomeric polymer, as defined in the first aspect of the invention, for providing elastic properties to a cosmetic composition.

According to a third aspect, the present invention relates to the use of a composition, as defined in the first aspect of the invention, for providing long-lasting hold, high-setting properties, and elasticity to the hair style.

According to fourth and fifth aspects, the present invention relates to methods for preparing a cosmetic composition, as defined in the first aspect of the invention.

According to a sixth aspect, the present invention relates to a method for applying a cosmetic composition, as defined in the first aspect of the invention, onto hair and/or skin.

As used herein, the term "elastomeric" means a group of polymers, which are cross-linked to only a certain degree in order to provide them with elasticity without making them too stiff. Said polymers usually are characterized with a glass transition temperature below room temperature. Said elastomers are classified as an intermediary category between non-cross-linked linear polymers and totally cross-linked polymers (e.g. thermosets).

As used herein, the expression "unit(s) (A)" means a repeat unit of one of the monomers constituting the polymer and comprising at least one carbon atom and its substituents and being polymerized with other units in order to form a polymeric chain. Said unit(s) is(are) derived from monomeric molecule(s) (A) - or monomer(s) (A).

As used herein, the expression "R₃" means an electron-withdrawing group enabling the break of the alpha-hydrogen bond.

As used herein, the expression "photoreactant" means a reactant which is activated by UV-rays so that to generate a pair of first radicals, said radicals abstracting then the hydrogen radical in alpha position relative to the electron-withdrawing group of the saturated polymeric backbone.

As used herein, the expression "direct covalent bond" means that the alpha carbon atoms of two different units (A) are bond to each other with a covalent bond by break of the carbon-hydrogen bond relative to R₃, i.e. said covalent bond comprises no intermediate molecules, wherein the expression "molecules" means at least one atom. Said "direct covalent bond", in the context of the present invention, is different from the covalent bond formed between two adjacent units during the polymerization process within one polymeric chain. In contrast, said "direct covalent bond" is formed in a second step (cross-linking step) after the polymer has been formed (polymerization step).

As used herein, the expression "polymer" means both homopolymers formed by polymerization of one type of monomers (A) and copolymers formed by polymerization of at least two types of monomers, a monomer (A) and a monomer (B). Copolymers can also comprise more than two comonomers. The expression "polymer" also means blends of at least one type of homopolymer with at least one other homopolymer, or copolymer, or blends of at least two copolymers. A polymer blend is **characterized in that** its glass transition temperature T_{g}^{B} equates to about the weighted average of the glass transition temperatures of its components. For example, the glass transition temperature of a polymer blend formed from 50% homopoylmer (A) and 50% homopolymer (B), T_{g}^{A,B}, will equate to about (T_{g}^{A} + T_{g}^{B})/2.

As used herein, the expressions "weight percentage" and "percentage by weight" of a component mean the percentage of active component and not the percentage of the raw material comprising this active, unless otherwise specified (see examples).

All percentages, ratios and proportions herein are by weight, unless otherwise specified.

### DETAILED DESCRIPTION OF THE INVENTION

According to a first aspect, the present invention relates to a cosmetic composition comprising, in a cosmetically acceptable carrier, elastomeric polymers comprising from 1% to 100% of polymerized units (A),
wherein said units (A), in their non-cross-linked form, comprise at least one carbon having an alpha-hydrogen relative to R₃ and have the general formula I 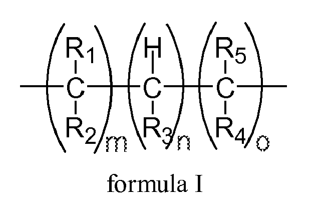
wherein R₃ is selected from the group consisting of a carboxyl derivative, a thiocarboxyl derivative a carbonyl derivative, a thiocarbonyl derivative, an imine derivative; a hydroxyl derivative; an aryl radical, a heteroaryl radical; a saturated cyclic radical; an alkoxy derivative; an amino derivative; an amide derivative; an imide derivative; a phosphorus derivative; a sulphur derivative; a tin derivative; a silica derivative; a alkyne derivative; a heterocyclic also incorporating R₂;
wherein R₁, R₂, R₄ and R₅ are the same or different and are selected from the groups consisting of hydrogen, C₁- C₂₂ saturated alkyl derivatives, C₁-C₂₂ unsaturated alkyl derivatives, hydroxyl derivatives, C1-C₂₂ alkoxy derivatives, carboxyl derivatives, a thiocarboxyl derivatives, amino derivatives, aminoalkyl derivatives, aminodialkyl derivatives, aminotrialkyl derivatives, tetraalkylammonium derivatives, amide derivatives, imide derivatives, sulfide derivatives, sulfoxide derivatives, sulfone derivatives, phosphite derivatives, phosphone derivatives, phosphonate derivatives, tin derivatives and silica derivatives, aryl derivatives, C₁-C₇ aryl derivatives, heteroaryl derivatives, C₁-C₇ heteroaryl derivatives, cyano derivatives, alkynyl derivatives;
wherein indicia m, o, n are independent to each other with m ≥ 0, o ≥ 0 and n ≥ 1; and,
wherein direct covalent bonds are formed between the alpha carbons of two units (A) by homolytic break of the alpha-hydrogen and wherein said bonds comprise no intermediate molecule.

The inventors have surprisingly found that elastomeric polymers - or elastomers - comprising some polymer segments bound to each other via direct covalent bonds (i.e. bonds not comprising intermediate molecule) exhibit satisfactory elastic properties. This differs from conventional cross-linking processes using cross-linking agents, which tend to alter/reduce the elastic properties of the cross-linked polymers. Without being bound by any theory, it is believed that the cross-linking technology as described herein may result in the obtaining of cross-linked polymers exhibiting a higher cross-linking density. Indeed, as the reaction undergoes via the radical hydrogen present in the saturated carbon-backbone of the polymer and therefore not only dependent upon the presence of a "functional" group within the polymer, the resulting cross-linking density would be therefore higher and the direct covalent bond would be distributed more evenly throughout the (co-)polymer than conventionally.

The inventors have also surprisingly found that cosmetic compositions - e.g. skin and/or hair care compositions, skin and/or hair cleansing compositions, hair styling compositions, hair conditioning compositions, hair dyeing compositions and/or shaving compositions - incorporating such polymers show an improved performance, particularly vis-à-vis the compositional properties, the ease of application and the benefits conferred to the skin and/or hair. Without being bound by any theory, it is believed that such improved performance results from the higher cross-linking density and from a more homogeneous distribution of direct covalent bonds throughout those newly formed elastomeric polymers. Specifically, such polymers are particularly suitable for incorporation into hair styling compositions. Said compositions confer long-lasting hold and high-setting properties, together with elasticity to the hair style.

When the polymeric chain comprises less than 100% of units (A), said chains further comprise units (B), said units (B) comprising no alpha-hydrogen and not being suitable for forming a direct covalent bond. Said units (B) may be, for example, methacrylic acid and derivatives. In one embodiment, the elastomeric polymers may comprise from 1 to 100% of polymerized units (A) and from 0% to 99% of polymerized units (B).

The elastomeric polymers comprise polymeric chains comprising from 1% to 100% of polymerized units (A). Said units (A) are derived from monomers (A), which are polymerized using conventional polymerization methods. The polymeric chains are preferably saturated chains.

Said units (A) comprise at least one carbon atom having an alpha-hydrogen relative to R₃ and have the below general formula I.

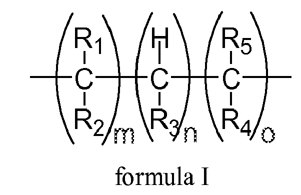

Units (A) comprise substituents R₃ being selected from the groups consisting of a carboxyl derivative, a thiocarboxyl derivative; carbonyl derivative, a thiocarbonyl derivative, an imine derivative; a hydroxyl derivative; an aryl radical, a heteroaryl radical; a saturated cyclic radical; an alkoxy derivative; an amino derivative, an amide derivative, an imide derivative; a phosphorus derivative; a sulphur derivative; a tin derivative; a silica derivative; a alkyne derivative; a heterocycle also incorporating R₂; or combinations thereof. Preferably, units (A) comprise substituents R₃ being selected from the groups consisting of a carboxyl derivative, carbonyl derivative, a thiocarbonyl derivative, an imine derivative; a hydroxyl derivative; an alkoxy derivative; an amino derivative; an amido derivative; or combinations thereof. More preferably, units (A) are selected from the groups consisting of acrylic acid, acrylamide, maleic acid, vinyl alcohol, vinyl acetate, vinyl methyl ether, vinyl pyrrolidone, vinyl-formamide, their derivatives, their salts, or combinations thereof. Still more preferably, units (A) are selected from the groups consisting of acrylic acid, acrylamide, vinyl alcohol, vinyl acetate, vinyl pyrrolidone, their derivatives, their salts, or combinations thereof

When R₃ is a carboxyl derivative, units (A) may be selected from the group consisting of acrylic acid, acrylamide, their derivatives, their salts, or combinations thereof. When R₃ is a hydroxyl derivative, units (A) may be selected from the group consisting of vinyl alcohol, its derivatives, their salts, or combinations thereof. When R₃ is an alkoxy derivative, units (A) may be selected from the group consisting of vinyl acetate, vinyl methyl ether, their derivatives, their salts, or combinations thereof. When R₃ is an amino derivative, units (A) may be selected from the group consisting of vinyl pyrrolidone, vinyl-formamide, their derivatives, their salts, or combinations thereof. When R₃ is part of a heterocycle also incorporating R₂, units (A) may be maleic acid, their derivatives, their salts, or combinations thereof.

Substituents R₁, R₂, R₄ and R₅ are independent to each other. R₁, R₂, R₄ and R₅ may be the same or different substituents. R₁, R₂, R₄ and R₅ are selected from the groups consisting of hydrogen, C₁-C₂₂ saturated alkyl derivatives, C₁-C₂₂ unsaturated alkyl derivatives, hydroxyl derivatives, C1-C₂₂ alkoxy derivatives, carboxyl derivatives, thiocarboxyl derivatives, amino derivatives, aminoalkyl derivatives, aminodialkyl derivatives, aminotrialkyl derivatives, tetraalkylammonium derivatives, amide derivatives, imide derivatives, sulfide derivatives, sulfoxide derivatives, sulfone derivatives, phosphite derivatives, phosphone derivatives, phosphonate derivatives, tin derivatives and silica derivatives, aryl derivatives, C₁-C₇ aryl derivatives, heteroaryl derivatives, C₁-C₇ heteroaryl derivatives, cyano derivatives, alkynyl derivatives. Alternatively, R₄ may be part of a heterocycle also incorporating R₂.

Indicia m, o, n are independent to each other with m ≥ 0, n ≥ 1 and o ≥ 0. Preferably, m = 0, o = 1 0 and n = 1.

The elastomeric polymers are obtained using a cross-linking process, different from the conventional cross-linking process using cross-linking agents and forming indirect covalent bonds. Said process comprises the step of forming direct covalent bonds between the alpha carbons of two different units (A) by break of the alpha hydrogen. Said direct covalent bonds differ from indirect covalent bonds in that they comprise no intermediate molecule. Conventional polymers may be cross-linked via direct covalent bonds, in order to obtain elastomeric polymers, prior to their incorporation into a cosmetic composition. Alternatively, conventional polymers may be cross-linked via direct covalent bonds after their incorporation into a cosmetic composition.

The direct covalent bonds may be formed between two units (A) within one polymeric chain - said bond being an intra-chain direct covalent bond. The direct covalent bonds may also be formed between two units (A) from two different polymeric chains - said bond being an inter-chain direct covalent bond.

Preferably, the direct covalent bonds are formed upon exposure of the polymers to ultraviolet irradiation at a wave length from 170 nm to 400 nm, from 0.001 sec to 22,000 sec, in the presence of a photoreactant agent in a ratio with units (A) from 0.01 to 1 and in the absence of a cross-linking agent. More preferably, the direct covalent bonds are formed at a temperature from 0°C to 95°C.

Alternatively, the direct covalent bonds are formed in the presence of an activated photoreactant agent in a ratio with units (A) from 0.01 to 1 and in the absence of a cross-linking agent. Preferably, the direct covalent bonds are formed at a temperature from 20°C to 70°C. The photoreactant agent may be activated upon exposure to ultraviolet irradiation at a wave length from 170 nm, from 0.001 sec to 22,000 sec.

As used herein, the expression "photoreactant" means a substance, which forms free radicals when being irradiated by light, especially by UV-light. The inventors have surprisingly found that, in order to form direct covalent bonds, it is essential to use a photoreactant agent present in an activated form and in a specific ratio (by weight) with units (A). The activation of the photoreactant agents is achieved upon their exposure to ultraviolet irradiation at a wave length from 170 nm to 400 nm, from 0.001 sec to 22,000 sec. The activation of the photoreactant agents and the formation of the direct covalent bonds may be concomitant. Alternatively, the activation of the photoreactant may be conducted prior forming the direct covalent bonds. Activating the photoreactant prior forming the direct covalent bonds is preferred as it prevents the polymers from being damaged by the irradiations. Using ultraviolet irradiation at a wave length from 170 nm to 400 nm is essential because the preferred photoreactant absorbs only UV-light. Using the photoreactant agents in a ratio (by weight) with units (A) from 0.01 to 1 is also essential in order to form direct covalent bonds. When using a ratio (by weight) of less than 0.01, it is expected no direct covalent bonds to be formed. Said ratio ranges preferably from 0.01 to 0.4, more preferably from 0.03 to 0.3.

The photoreactant agent may be a persulfate salt. Preferably, the persulfate salts are selected from the group consisting of sodium persulfate, potassium persulfate, ammonium persulfate or mixtures thereof. Using persulfate salts is preferred because they are easy to handle, highly soluble in water and form with each homolytic cleavage two equal radicals at the same time.

From 0.01% to 100% of units (A) may be cross-linked via direct covalent bonds. Preferably, from 0.1% to 50% of units (A) are cross-linked. More preferably, from 1% to 5% of units (A) are cross-linked. When only part of the units (A) are cross-linked via direct covalent bonds, the elastomeric polymers comprise units (A) in a cross-linked form and units (A) in an uncross-linked form. The degree of elasticity varies depending on the degree of cross-linking, i.e. the proportion of units (A) being cross-linked.

Any polymers conventionally used in cosmetic compositions, comprising from 1% to 100% of units (A) comprising at least one carbon having an alpha-hydrogen relative to R₃, are suitable for cross-linking via direct covalent bonds and for incorporation into the cosmetic composition of the present invention.

Said polymers may be uncross-linked polymers or cross-linked polymers using conventional cross-linking processes using cross-linking agents. When said polymers are cross-linked polymers, they could be further cross-linked according to the present method in order to create direct covalent bonds and to obtain elastomeric polymers. In such case, these elastometic polymers would comprise "indirect covalent bonds" (i.e. conventional cross-links) and "direct covalent bonds".

Said polymers may be emulsion stabilizers, suspending agents, viscosity increasing agents, opacifying agents, film-forming agents, hair-fixative agents, skin moisturizing agents and/or hair conditioning agents. Preferably, said polymers are hair-fixative agents - also called hair styling polymers. Said polymers may be selected from the group consisting of anionic polymers, cationic polymers, amphiphilic polymers, non-ionic polymers or combinations thereof. See polymers listed in the International Cosmetic Ingredient Dictionary and Handbook, 12^{th} edition (2008) from the Personal Care Products Council (formerly the Cosmetic, Toiletry and Fragrance Association or CTFA). Suitable anionic polymers may be polyacrylic acid, acrylate/t-butylacrylamide copolymer (commercially available under the tradename Ultrahold 8 from BASF corporation), AMP-acrylates copolymer (e.g. DynamX from AzkoNobel Surface Corporation), VA/crotonates copolymers (e.g. Luviset CA from Clariant International Ltd.), isobutylene/ethylmaleimide/hydroxyethylmaleimide copolymer (e.g. Aquaflex FX 64 from International Specialty Products), PVM/MA copolymers being copolymers of methyl vinyl ether and maleic anhydride or maleic acid (e.g. Gantrez AN-119/139/149/169, S-95/97 from International Specialties Products), ethyl (or butyl, or propyl) ester of PVM/MA copolymers (e.g. Gantrez A-4259/ES 425/ES 435/V-425 from International Specialties Products), acrylamide/sodium acryloyldimethyltaurate/acrylic acid copolymer (e.g. Acudyne SCP from Rohms and Haas Company, Inc.), acrylates/hydroxyesters acrylates copolymer (e.g. Acudyne DHR from Rohms and Haas Company, Inc.). Suitable cationic polymers may be polyquaternium -11, polyquaternium-16, and polyquaternium-68. Suitable amphiphilic polymers may be octylacrylamide/acrylates/butylaminoethyl methylacrylate copolymer (e.g. Amphomer from AkzoNobel Surface Company), poly-vinyl alcohol (e.g. celvol from Celanese Chemicals), vinyl pyrrolidone/vinyl acetate copolymers (e.g. Luviskol VA-37E/73E/64 Powder/64W/73W from BASF Corporation), polyvinyl methyl ether (e.g. Gantrez M-154 from International Specialties Products), polyvinylpyrrolidine (e.g. Luviskol K30/K90/K17/K80/K90 from BASF Corporation), polyvinylformamide, sodium polystyrene sulfonate (e.g. Flexan II from AkzoNobel Company).

The inventor has surprisingly found that cross-linking polymers conventionally used in cosmetic compositions - as those listed above - allows improving/conferring elastic properties of/to these polymers. For example, polyacrylic acid, polyvinyl pyrrolidone, or polyvinyl acetate cross-linked via direct covalent bonds shows superior elastic properties versus conventional polyacrylic acid, polyvinyl pyrrolidone, or polyvinyl acetate - such conventional polymers being either not cross-linked or cross-linked via indirect covalent bond (i.e. using cross-linking agents). By incorporating such polymers into cosmetic compositions, improved performances are achieved, particularly vis-à-vis the compositional properties, the ease of application and the benefits conferred to the skin and/or hair. Particularly, by incorporating such polymers into hair-styling compositions, it confers a satisfactory elasticity to the hair style. For example, the elasticity of a hair style may be assessed using the curl retention test.

The compositions, according to the present invention, also comprise a cosmetically acceptable carrier. Said carrier may be selected from solutions, emulsion, solid carrier, dispersion, suspension, mousses, gels, waxes, polyphasic compositions.

The compositions, according to the present invention, may be applied onto the skin, including the forehead, the cheeks and neck, the arms and the legs, as well as onto keratinous fibers, including hair and facial hair. Said compositions may be applied as a leave-on composition or as a rinse-off composition.

The composition, according to the present invention, may further comprise a cosmetic ingredient, preferably, from 0,001% to 50% of at least one cosmetic ingredient. Preferably, said cosmetic active ingredients are selected from the groups consisting of emulsifiers/surfactants; structuring agents; non-elastomeric thickening agents; anti-oxidants/radical scavengers; chelators and sequestrants; desquamation agents/exfoliants; skin lightening agents; skin and hair conditioning agents; hair-styling agents; anti-frizz agents; anti-wrinkle actives/anti-atrophy agents; anti-cellulite agents; tanning agents; anti-acne agents; skin freshening, skin soothing and skin healing actives; anti-inflammatory agents; cleansing agents; lathering agents; beard wetting agents; antimicrobial, antibacterial and antifungal actives; sunscreen actives and sunblocks agents; shine enhancing agents, viscosity modidiers; pH adjusting agents; solid particulates; humectants and emollients; electrolytes; vitamins, their derivatives, and their salts thereof; natural extract and their derivative thereof; peptides; colorants; preservatives; fragrances and perfumes; propellants, or mixtures thereof. More preferably, said cosmetic active ingredients are ingredients suitable for incorporation into a hair styling composition.

The compositions, according to the present invention, may be selected from skin care composition or hair care compositions. Preferably, the compositions are skin cleansing, hair cleansing, hair styling, hair conditioning, hair dyeing and/or shaving compositions, or combinations thereof. More preferably, said compositions are hair styling compositions.

The inventors have surprisingly found that the incorporation of elastomeric hair styling polymers into hair styling compositions is particularly beneficial as said compositions exhibit improved performance when applied onto hair. Indeed, said compositions confer long-lasting, high setting properties as well as elasticity to the hair style. The elasticity confers to the hair style is superior to the elasticity conferred to hair when applying a hair styling composition incorporating conventional hair styling polymers, i.e. polymers not being cross-linked via direct covalent bond.

According to a second aspect, the present invention relates to the use of the elastomeric polymer, as defined above for providing elastic properties to a cosmetic composition. The inventors have effectively surprisingly found that the use of such elastomeric polymers - versus commercially available polymers not comprising direct covalent bonds - improves the elasticity properties of cosmetic compositions.

According to a third aspect, the present invention relates to the use of a composition, as defined above, for providing long-lasting hold, high-setting properties, and elasticity to the hair style. The inventors have effectively surprisingly found that hair styling compositions comprising elastomeric polymers comprising direct covalent bond are useful for imparting a satisfactory elasticity to the hair style.

According to a fourth aspect, the present invention relates to a method for preparing a cosmetic composition, as defined in the first aspect of the invention, said method comprising the steps of:
(a) providing a polymer comprising from 1% to 100% of polymerized units (A);
(b) forming direct covalent bonds between the alpha carbons of two different units (A) of said polymers by break of the alpha-hydrogen-bond relative to R₃, said bonds comprising no intermediate molecule, in order to obtain elastomeric polymers;
(c) providing a cosmetic composition comprising a cosmetically acceptable carrier and optionally at least one cosmetic active ingredient;
(d) incorporating said elastomeric polymers into said cosmetic composition.

Step (b) may be carried out by exposing said polymers to ultraviolet irradiation at a wavelength from 170 nm to 400 nm, from 0.001 sec to 22,000 sec, in the presence of a photoreactant agent in a ratio with units (A) from 0.01 to 1 and in the absence of a cross-linking agent, and optionally at a temperature from 0°C to 95°C.

Alternatively, step (b) may comprise several sub-steps so that (b1) providing a photoreactant, then (b2) activating said photoreactant upon exposure to ultraviolet irradiation at a wavelength from 170 nm, from 0.001 sec to 22,000 sec, then (b3) mixing said polymers and said activated photoreactant in a ratio with units (A) from 0.01 to 1 and in the absence of a cross-linking agent in order to form direct covalent bonds.

Suitable polymers comprising from 1% to 100% of polymerized units (A), photoreactants, cosmetically acceptable carriers, and optional cosmetic active ingredients are detailed hereinbefore.

According to a fifth aspect, the present invention relates to a method for preparing a cosmetic composition, as defined in the first aspect of the invention, said method comprising the steps of:
(a) providing a cosmetic composition comprising a cosmetically acceptable carrier, and optionally at least one cosmetic active ingredient;
(b) incorporating polymers comprising from 1% to 100% of polymerized units (A) into said cosmetic composition;
(c) forming direct covalent bonds between the alpha carbons of two different units (A) of said polymers by break of the alpha-hydrogen-bond relative to R₃, said bonds comprising no intermediate molecule, in order to obtain elastomeric polymers.

Step (c) may be carried out by exposing said composition - and therefore said polymers - to ultraviolet irradiation at a wavelength from 170 nm to 400 nm, from 0.001 sec to 22,000 sec, in the presence of a photoreactant agent in a ratio with units (A) from 0.01 to 1 and in the absence of a cross-linking agent, and optionally at a temperature from 0°C to 95°C.

Alternatively, step (c) may comprise several sub-steps so that (c1) providing a photoreactant, then (c2) activating said photoreactant upon exposure to ultraviolet irradiation at a wavelength from 170 nm, from 0.001 sec to 22,000 sec, then (c3) mixing said polymers and said activated photoreactant in a ratio with units (A) from 0.01 to 1 and in the absence of a cross-linking agent in order to form direct covalent bonds.

Suitable polymers, photoreactants, cosmetically acceptable carriers, and optional cosmetic active ingredients are detailed hereinbefore.

In a sixth aspect, the present invention relates to a method for applying a cosmetic composition onto hair and/or skin, said method comprising the steps of:
(a) providing a cosmetic composition, as defined in the first aspect of the invention comprising, in a cosmetically acceptable carrier, elastomeric polymers comprising from 1% to 100% of polymerized units (A) wherein direct covalent bonds are formed between the alpha carbons of two different units (A) by break of the alpha-hydrogen-bond relative to R₃ and wherein said bonds comprise no intermediary molecule, and optionally at least one cosmetic active ingredient;
(b) applying said composition onto skin and/or hair

Said composition may be obtained by any suitable methods, particularly with the methods according to the fourth and fifth aspects of the invention as detailed hereinbefore.

### Examples

The following examples further describe and demonstrate the preferred embodiments within the scope of the present invention. The examples are given solely for the purpose of illustration, and are not to be construed as limitations of the present invention since many variations thereof are possible without departing from its scope.

All weights provided in these examples are weights of the commercially available materials, including active(s) and/or solvent and/or by-products.

### Example 1 - "direct cross-linking" of polymers - general instructions

To 1000 ml of a 5% to 10% solution of said polymer (MW ≈ 50 000 to 200 000 g/mol), 10% to 15% by weight of polymer of ammoniaperoxodisulfate is added. The temperature is 25°C to 85°C. The sample is stirred and irradiated so that the emission spectrum comprises UV light between 220 nm and 300 nm. After 1 to 60 minutes of irradiation, "direct" cross-links are formed. In case the polymer comprises both units (A) and units (B), the ratio pertains to the ratio of photoreactant agents to units (A).

### Example 2 - "direct cross-linking" of poly-vinyl alcohol

a) To 1000 ml of a 5% solution of poly vinyl alcohol (MW ≈ 200 000 g/mol, degree of hydrolysis ≥ 85%), ammoniaperoxodisulfate (= 20 % of the amount of polymer) was added. The temperature was 50 °C. The sample was stirred and irradiated by a submerse middle-pressure mercury lamp. The emission spectrum comprises UV light between 220 nm and 300 nm. After 10 minutes of irradiation, the former thin solution changed into a thick, viscous gel.
b) To 1000 ml of a 5% (by weight) solution of poly vinyl alcohol (MW ≈ 200 000 g/mol, degree of hydrolysis ≥ 85%), 10 g of ammoniaperoxodisulfate (= 20 % of the amount of polymer) was added. The temperature was 50°C. The sample was stirred and irradiated by a submerse low-pressure mercury lamp. The emission spectrum comprises monochromatic UV light of 253.7 nm. After 20 minutes of irradiation, the former thin solution changed into a thick, viscous gel.

### Example 3 - "direct cross-linking" of poly-vinyl pyrrolidone

a) To 1000 ml of a 5% solution of poly vinyl pyrrolidone (Luviskol K 90), ammoniaperoxodisulfate (= 10 % by weight of the amount of polymer) was added. The temperature was 50°C. The sample was stirred and irradiated by a submerse middle-pressure mercury lamp. The emission spectrum comprises UV light between 220 nm and 300 nm. After 20 minutes of irradiation, the former thin solution changed into a thick, viscous gel.
b) To 1000 ml of a 5% (by weight) solution of poly vinyl pyrrolidone (Luviskol K 90), 5 g of ammoniaperoxodisulfate (= 10 % of the amount of polymer) was added. The temperature was 50°C. The sample was stirred and irradiated by a submerse low-pressure mercury lamp. The emission spectrum comprises monochromatic UV light of 253.7 nm. After 30 minutes of irradiation, the former thin solution changed into a thick, viscous gel.

### Example 4 - "direct cross-linking" of poly-acrylic acid

a) To 1000 ml of a 15% (by weight) solution of poly acrylic acid (MW ≈ 100000 g/mol), ammoniaperoxodisulfate (= 10 % by weight of the amount of polymer) was added. The temperature was 50°C. The sample was stirred and irradiated by a submerse middle-pressure mercury lamp. The emission spectrum comprises UV light between 220 nm and 300 nm. After 50 minutes of irradiation, the former thin solution changed into a thick, viscous gel.
b) To 1000 ml of a 15% (by weight) solution of poly acrylic acid from Aldrich (MW ≈ 100000 g/mol), 15 g of ammoniaperoxodisulfate (= 10 % (by weight) of the amount of polymer) was added. The temperature was 50°C. The sample was stirred and irradiated by a submerse low-pressure mercury lamp. The emission spectrum comprises monochromatic UV light of 253.7 nm. After 70 minutes of irradiation, the former thin solution changed into a thick, viscous gel.

### Example 5 -

The "direct cross-linking" of further commercially available polymers - including Luviset Clear (VP/methacrylamide/vinyl imidazole copolymer) from BASF Corporation; Acudyne SCP (acrylamide/sodium acryloyldimethyltaurate/acrylic acid copolymer) from Rohm and Haas Company Inc.; Luviset CA66 (VA/crotonates copolymer) from BASF Corporation; Aquaflex SF-40 (VP/vinyl caprolactam/DMAPA acrylates copolymer) from International Specialty Products; polyquaternium-11; Aquaflex FX 64 (isobutylene/ethylmaleimide/hydroxyethylmaleimide copolymer) from International Specialty Products; Hair Gloss Polymer A (octylacrylamide/acrylates/butylaminoethyl methacrylate copolymer) from Cosmetochem International AG; Coplymer VC-173 (Vinyl Caprolactam/VP/dimethylaminoethyl methacrylate copolymer) from International Specialty Products; Luvimer 100P (acrylates copolymer) from BASF Corporation - was also successfully conducted using a similar methods as those of examples 1 to 3.

### Example 6 - Measurement of elastic properties - DMA Test of Poly(vinylalcohol)

General - Dynamic Mechanical Analysis (DMA herein) is a known method for mechanical material characterization of films. DMA is using oscillatory principle for characterization of mechanical properties of solid materials (e.g. films). The sample is fixed in clamps of the instrument and stress is applied in vertical direction. The ratio of the Loss Modulus G" and the Storage Modulus G' is called tan δ and is obtained with the equation tan δ = G"/ G'. tan δ is known as a measure of the elasticity of a sample - the lower tan δ is, the higher the elastic fraction of the system.

Samples preparation - It is provided a poly(vinylalcohol) from Merck. Each batch (20 ml of the polymer solution with photoreactant) is put into an open form with glass bottom and then irradiated with UV light using medium pressure UV-lamp TQ 150, Heraeus) placed at a distance of 20cm from the batch. After exposure to UV light, the batches are dried for five days in order to obtain a film. Samples of 10mm×5,3mm×0,3mm were cut out from said film in order to conduct DMA measurements.

DMA test parameters: DMA measurements were conducted isothermally using a TA Instruments DMA Q800 at 23°C and constant humidity (50%), with a frequency sweep from 1 to 100 Hz with 0,03% strain.

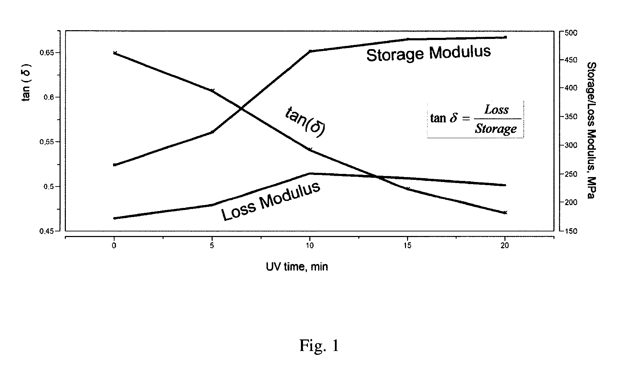

Results: Data obtained are shown in figure 1, which shows tan δ value function of the UV time (min). As can be extrapolated from figure 1, the decrease of tan δ value - and therefore the increase of the elasticity (elastic properties) of the elastomeric polymers - is directly correlated with the increase exposure of the batch to UV light.

### Example 7 - Measurements - UV-Rheology of Poly(vinylpyrrolidone)

General: UV-Rheology measurements are conducted using TA Instruments AR-2000ex in order to measure the rheological properties of batches of polymers (G', G", tan δ) during exposure to UV light (i.e. while the cross-linking occurs). This allows therefore a real-time monitoring of viscoelastic properties changes occurring before, during and after UV irradiation.

Samples preparation: Batches comprising 20% solution of PVP (Luviskol K 90, BASF) are prepared. Such batches comprise different proportions of photoreactant (from 0 to 40% of the polymer mass). Said photoreactant is added to the batches just before conducting the measurements in order to prevent early cleavage.

UV-Rheology test parameters: Rheology experiments are conducted isothermally at two different temperatures (i.e. 25°C and 40°C), using a 20mm steel plate and setting the gap to 200 µm About 1 ml of polymer solution is used. Measurements were carried out as time sweep using strain control 5% with lower torque limit of 10 µN.m and a frequency of 0,3Hz. All along the measurements, the polymer solution was exposed to UV light using ProCure PLG-10, medium pressure UV-lamp ES270.

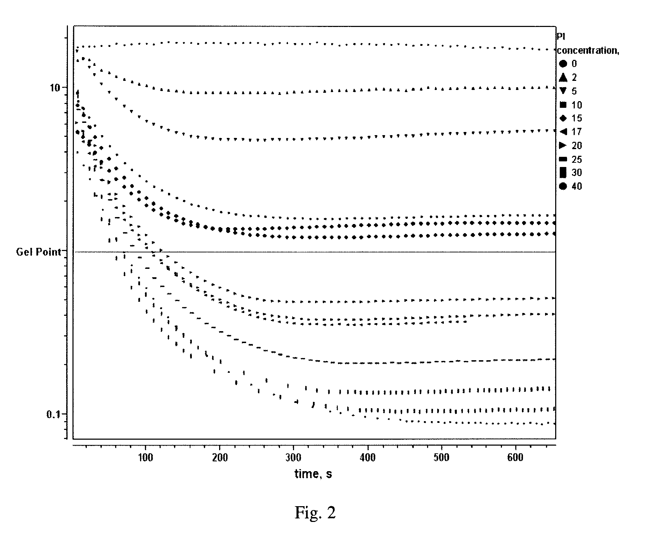

Results: data obtained are shown in figure 2 output of UV-Rheology tests is the behaviour of tan δ (= loss modulus / storage modulus). Based on the results assumptions on physical and chemical nature of cross-linking process can be made.

The examples below disclose cosmetic compositions - according to the present invention - comprising at least one elastomeric polymer (or directly cross-linked polymer - see symbol "*"). Said compositions may also comprise further ingredients, such as from 0,1 to 0,3g fragrance:
Example 8 (liquid gel): 0.3g elastomeric poly(acrylic acid) *; 1.5g PEG-12 dimethicone; 0.3g aminoethyl propanol 95 %; 0.2g PPG-1 / PEG-9 lauryl glycol ether; 0,4g hydroxyethylcellulose; 16,5g ethanol; qsp 100g with water.
Example 9 (liquid gel): 1.0g elastomeric poly(vinylpyrrolidone) *; 1.0g PEG-12 dimethicone; 1,2g xantham gum; 0.1g citric acid; 6.5g ethanol; 0.3g DMDM hydantoin; qsp 100g with water.
Example 10 (liquid gel): 0,5 g elastomeric poly(vinylalcohol) *; 7,0 g glucose; 3,8 g propylenglycol; 0,3 g hydroxypropylguar; 0,2 g aminomethyl propanol 95 %; 0,5 g PEG-25 PABA; 0,18 g PEG-40 hydrogenated castor oil; 0,18 g PPG-1 / PEG-9 lauryl glycol ether; 16,5 g ethanol; qsp 100g with water.
Example 11 (spray gel): 3,0g elastomeric poly(vinylpyrrolidone) *; 18g ethanol; 0,1g aminomethyl propanol 95%; 0,2g PEG-40; 0,5g acrylates / beheneth-25 methacrylate copolymer; 70g water.
Example 12 (Gel with short drying time): 2,8g elastomeric poly(acrylic acid) *; 1,5g PEG-12 dimethicone; 0,35g acrylates / C10-30 alkyl acrylate crosspolymer; 0,26g aminomethyl propanol 95 %; 0,30g methylmethoxycinnamate; 34,2g ethanol; qsp 100g with water.
Example 13 (gel-spray): 0,23g elastomeric poly(acrylic acid) *; 1,0g PEG-12 dimethicone; 0,22g aminoethyl propanol 95 %; 6,5g ethanol; qsp 100g with water.
Example 14 (blow dryer-gel): 1,0g elastomeric acrylamide / sodium acryloyldimethyltaurate / acrylic acid copolymer *; 1,0g PEG-12 dimethicone; 0,95g hydroxypropylcellulose; 0,1g citric acid; 6,5g ethanol; qsp 100g with water.
Example 15 (gel): 1,8g elastomeric poly(vinylpyrrolidone) [Luviskol K 90] *; 1,5g PEG-12 dimethicone; 1,0g acrylates / palmeth-25 acrylate copolymer; 0,3g aminomethyl propanol 95%; 0,3g PEG-25 PABA; 0,15g d-panthenol; 34,2g ethanol; 0,1g hydrolyzed hair keratin; qsp 100g with water.
Example 16 (gel): 2,5g elastomeric VA / crotonates copolymer *; 4,2g sorbitol; 0,8g carbomer; 0,3g aminomethyl propanol 95 %; 0,2g methylparabene; 0,2g PEG-40 hydrogenated castor oil; 0,1g d-panthenol; 5,0g ethanol; qsp 100g with water.
Example 17 (gel): 1,5g elastomeric VP / vinyl caprolactam / DMAPA acrylates copolymer *; 1,2g vinyl acrylate / crotonic acid copolymer; 4,2g sorbitol; 0,12g acrylates / ceteth-20 itaconate copolymer; 0,35g aminomethyl propanol 95%; 0,5g PEG-25 PABA; 0,2g DMDM hydantoin; 0,2g PEG-40 hydrogenated castor oil; 0,1g d-panthenol; 5,0g ethanol; qsp 100g with water.
Example 18 (gel): 5,2g glycerine; 4,0g propylene glycol; 0,35g elastomeric poly(acrylic acid) *; 0,26g aminomethyl propanol 95%; 1,0g polysorbate 40; 0,2g methylparabene; 0,5g PEG-25 PABA; 4,5g ethanol; qsp 100g with water.
Example 19 (pump-setting mousse): 0,3g elastomeric vinylacetate / crotonic acid copolymer; 0,4g cocamidopropyl hydroxysultaine; 0,1g citric acid; 8,9g ethanol; 0,1g betaine; qsp 100g with water.
Example 20 (pump-setting mousse): 0,4g elastomeric acrylic acid / ethylacrylate / N-tert-butylacrylamide copolymer *; 0,4g cocamidopropyl hydroxysultaine; 0,1g citric acid; 0,2g DMDM hydantoin; 0,1g *Chamomilla Recutita (Matricaria)* flower extract; qsp 100g with water.
Example 21 (pump-setting mousse): 1,2g elastomeric VP / methacrylamide / vinyl imidazole copolymer *; 0,35g polyquaternium-6; 0,4g cocamidopropyl hydroxysultaine; 0,1g d-panthenol; 8,9g ethanol; 0,1g betaine; qsp 100g with water.
Example 22 (pump-setting mousse): 2,5g elastomeric VP/methacrylamide/vinyl imidazole copolymer *; 8,9g ethanol; 0,2g cocamidopropyl hydroxysultaine; 0,2g cetyltrimethylammoniumchloride; 0,1g Silkpro Liquid; qsp 100g with water.
Example 23 (pump-setting mousse): 2,0g elastomeric VP / methacrylamide / vinyl imidazole copolymer *; 0,3g polyquaternium-4; 8,9g ethanol; 0,2g cocamidopropyl hydroxysultaine; 0,2g cetyltrimethyl ammonium chloride; 0,1g citric acid; 0,1g betaine; qsp 100g with water.
Example 24 (pump-setting mousse): 0,3g elastomeric polyquaternium-11 *; 0,4g cocamidopropyl hydroxysultaine; 1,0g propylenglycol; 0,2g methylparabene; qsp 100g with water.
Example 25 (pump-setting mousse): 1,8g elastomeric VP / methacrylamide / vinylimidazole copolymer *; 0,4g cocamidopropyl hydroxysultaine; 0,1g *Rosmarinus Officinalis* (rosemary) leaf extract; 8,9g ethanol; 0,1g D-panthenyl ethylether; qsp 100g with water.
Example 26 (aerosol setting mousse): 0,5g elastomeric isobutylene / ethylmaleimide / hydroxyethylmaleimide copolymer *; 4,0g butane; 4,0g propane; 8,9g ethanol; 0,4g PEG 25 PABA; 0,15g betaine; 0,2g laureth-4; 0,05g cetrimonium bromide; 0,5g amodimethicone; qsp 100g with water.
Example 27 (aerosol setting mousse): 1,5g elastomeric VP / methacrylamide / vinyl imidazole copolymer *; 0,5g polyquaternium-47; 4,0g butane; 4,0g propane; 0,15g betaine; 0,25g mixture of cyclopentasiloxane, cyclotetrasiloxane, dimethiconol; 0,2g ethylhexyl methoxycinnamate; 0,2g laureth 4; 0,07g cetrimonuim chloride; qsp 100g with water.
Example 28 (aerosol setting mousse): 2,1g elastomeric VP / methacrylamide / vinyl imidazole copolymer *; 2,5g VP / dimethylamino ethylmethacrylate copolymer; 1,0g polyquaternium-4; 4,0g butane; 4,0g propane; 0,2g panthenol; 0,7g quaternium-80; 0,07g cetrimonuim chloride; qsp 100g with water.
Example 29 (aerosol setting mousse): 2,1g elastomeric VP / methacrylamide / vinyl imidazole copolymer *; 0,6g elastomeric vinylacetate / crotonic acid copolymer *; 0,5g polyquaternium-7; 4,0g butane; 4,0g propane; 8,9g ethanol; 0,4g PEG-25 PABA; 0,2g panthenol; 0,2g laureth 4; 0,07g C-11 pareth-8; qsp 100g with water.
Example 30 (setting lotion for spraying): 1,5g elastomeric VP / methacrylamide / vinyl imidazole copolymer *; 1,0g isobutylene / ethylmaleimide / hydroxyethyl maleimide copolymer; 2,7g ethanol; 1,0g polyquaternium-35; 0,7g PEG-25 PABA; 0,35g panthenol; 0,2g cetrimonuim chloride; 0,21g PEG-40 hydrogenated castor oil; qsp 100g with water.
Example 31 (setting lotion for spraying): 2,0g elastomeric octylacrylamide / acrylates / butylamino ethylmethacrylate copolymer *; 28,5g ethanol; 0,6g aminomethyl propanol 95%; 0,20% cetyltrimethyl ammonium bromide; qsp 60 water.
Example 32 (setting lotion for spraying): 0,65g elastomeric octylacrylamide / acrylates / butylamino ethylmethacrylate copolymer *; 0,2g polyquaternium-4; 28,5g ethanol; 0,6g aminomethyl propanol 95%; 0,2g cetyltrimethylammonium chloride; qsp 60g water.
Example 33 (non-aerosol air blower lotion): 2,0g elastomeric vinyl caprolactam / VP / dimethylamino ethylmethacrylate copolymer *; 28,5g ethanol; 0,20g cetyltrimethyl ammonium Chloride; qsp 60g water.
Example 34: (non-aerosol air blower lotion): 3,1g elastomeric VP / methacrylamide / vinyl imidazole copolymer *, 0,05g polyquaternium-4, 0,5g acrylates / lauryl acrylate / stearyl acrylate / ethylamine oxide methacrylate copolymer, 27g ethanol, 0,1g betain, 0,21g PEG 40 hydrogenated castor oil, 0,20g cetyltrimethyl ammonium Bromide, qsp 100g with water.
Example 35 (aerosol air blower lotion): 3,0g elastomeric VP / methacrylamide / vinyl imidazole copolymer *; 2,30g sodium polystyrene sulfonate; 0,02g phenyl trimethicone; 10,0g water; qsp 100g with ethanol. Said lotion is packaged together with Dimethylether in the ration 45%/55%.
Example 36 (VOC 80 pump spray): 0,5g t-elastomeric butylacrylate / ethylacrylate / methacrylic acid copolymer *; 0,10g aminomethyl propanol; 0,05g betain; 55g ethanol; qsp 100g with water.
Example 37 (aerosol hairspray): 10,0g water; 0,02g phenyl trimethicone; 1,5g elastomeric VP / methacrylamide / vinyl imidazole copolymer *; 3,00g octylacrylamide / acrylic acid / butyl amino ethyl methacrylate / methyl methacrylate / hydroxyl propyl methacrylate copolymer; 0,48g aminomethyl propanol 95%; qsp 100g with ethanol. Said hairspray is packaged together with dimethylether in the ration 45%/55%.
Example 38 (aerosol hairspray): 10,0g water; 3,3g elastomeric t-butylacrylate / ethyl acrylate / methacrylic acid copolymer *; 0,84g aminomethyl propanol 95%; 1,0g VA / crotonates copolymer; qsp 100g with ethanol. Said hairspray is packaged together with dimethylether in the ration 45%/55%.
Example 39 (aerosol hairspray): 2,50g elastomeric VP / methacrylamide / vinyl imidazole copolymer *; 3,30g t-butylacrylate / ethylacrylate / methacrylic acid copolymer; 0,85g amino methyl propanol 95%; 0,02g phenyl trimethicone; 10,0g water; qsp 100g with ethanol. Said hairspray is packaged together with dimethylether in the ration 45%/55%.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A cosmetic composition comprising, in a cosmetically acceptable carrier, elastomeric polymers comprising from 1% to 100% of polymerized units (A),
wherein said units (A), in their non-cross linked form, comprise at least one carbon having an alpha-hydrogen relative to R₃ and have the general formula I 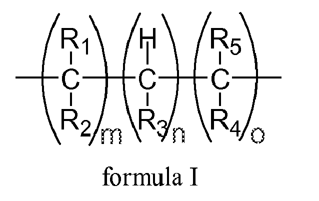 wherein R₃ is selected from the group consisting of:
(a) a carboxyl derivative, a thiocarboxyl derivative;
(b) a carbonyl derivative, a thiocarbonyl derivative, an imine derivative;
(c) a hydroxyl derivative
(d) an aryl radical, a heteroaryl radical;
(e) a saturated cyclic radical;
(f) an alkoxy derivative;
(g) an amino derivative, an amide derivative, an imide derivative;
(h) a phosphorus derivative;
(i) a sulphur derivative;
(j) a tin derivative;
(k) a silica derivative;
(l) a alkyne derivative;
(m)a heterocyclic also incorporating R₂;
wherein R₁, R₂, R₄, and R₅ are the same or different and are selected from the groups consisting of hydrogen, C₁-C₂₂ saturated alkyl derivatives, C₁-C₂₂ unsaturated alkyl derivatives, hydroxyl derivatives, C1-C₂₂ alkoxy derivatives, carboxyl derivatives, thiocarboxyl derivatives, amino derivatives, aminoalkyl derivatives, aminodialkyl derivatives, aminotrialkyl derivatives, tetraalkylammonium derivatives, amide derivatives, imide derivatives, sulfide derivatives, sulfoxide derivatives, sulfone derivatives, phosphite derivatives, phosphone derivatives, phosphonate derivatives, tin derivatives and silica derivatives, aryl derivatives, C₁-C₇ aryl derivatives, heteroaryl derivatives, C₁-C₇ heteroaryl derivatives, cyano derivatives, alkynyl derivatives;
wherein indicia m, o, n are independent to each other with m ≥ 0, o ≥ 0 and n ≥ 1; and,
wherein direct covalent bonds are formed between the alpha carbons of two different units (A) by break of the alpha-hydrogen-bond relative to R₃ and wherein said bonds comprise no intermediate molecule.

2. A composition, according to claim 1, wherein said covalent bonds are formed upon exposure of said polymers to ultraviolet irradiation at a wave length from 170 nm to 400 nm, from 0.001 sec to 22,000 sec, preferably at a temperature from 0°C to 95°C, in the presence of a photoreactant agent in a ratio with units (A) from 0.01 to 1 and in the absence of a cross-linking agent.

3. A composition, according to claim 1, wherein said covalent bonds are formed, preferably at a temperature from 0°C to 95°C, in the presence of an activated photoreactant agent in a ratio with units (A) from 0.01 to 1 and in the absence of a cross-linking agent.

4. A composition, according to claim 3, wherein said photoreactant agent is activated upon exposure to ultraviolet irradiation at a wave length from 170 nm to 400 nm, from 0.001 sec to 22,000 sec.

5. A composition, according to any of the preceding claims, wherein said covalent bonds are formed prior to the incorporation of the elastomeric polymers into said cosmetic composition.

6. A composition, according to any of claims 2 to 5, wherein said photoreactant is a persulfate salt, preferably said persulfate salt is selected from the groups consisting of sodium persulfate, potassium persulfate, ammonium persulfate or mixtures thereof.

7. A composition, according to any of the preceding claims, wherein said units (A) comprise substituents R₃ being selected from the groups consisting of:
(a) a carboxyl derivative, a carbonyl derivative, a thiocarbonyl derivative, an imine derivative;
(b) a hydroxyl derivative
(c) an alkoxy derivative;
(d) an amino derivative or amido derivative; or combinations thereof.

8. A composition, according to any of the preceding claims, wherein the indicia are m= 0, o = 0, n =1.

9. A composition, according to claim 7, wherein said units (A) are selected from the groups consisting of acrylic acid, acrylamide, maleic acid, vinyl alcohol, vinyl acetate, vinyl methyl ether, vinyl pyrrolidone, vinyl-formamide, their derivatives, their salts, or combinations thereof.

10. A composition, according to any of the preceding claims, wherein the cosmetically acceptable carrier is selected from the group consisting of solution, emulsion, solid carrier, suspension, dispersion, mousse, gel, wax or polyphasic composition

11. A composition, according to any of the preceding claims, wherein said composition comprises a cosmetic ingredient, preferably a cosmetic active ingredient being selected from the group consisting of emulsifiers/surfactants; structuring agents; non-elastomeric thickening agents; anti-oxidants/radical scavengers; chelators and sequestrants; desquamation agents/exfoliants; skin lightening agents; skin and hair conditioning agents; hair-styling agents; anti-frizz agents; anti-wrinkle actives/anti-atrophy agents; anti-cellulite agents; tanning agents; anti-acne agents; skin freshening, skin soothing and skin healing actives; anti-inflammatory agents; cleansing agents; lathering agents; beard wetting agents; antimicrobial, antibacterial and antifungal actives; sunscreen actives and sunblocks agents; shine enhancing agents, viscosity modidiers; pH adjusting agents; solid particulates; humectants and emollients; electrolytes; vitamins, their derivatives, and their salts thereof; natural extract and their derivatives thereof; peptides; colorants; preservatives; fragrances and perfumes; propellants; or mixtures thereof.

12. A composition, according to any of the preceding claims, wherein said composition is selected from the group consisting of skin cleansing, hair cleansing, hair styling, hair conditioning, hair dyeing and/or shaving compositions, or combinations thereof, preferably said composition is a hair styling composition.

13. The use of the elastomeric polymer, as defined in any of the preceding claims for providing elastic properties to a cosmetic composition.

14. The use of a composition, as defined in any of claims 1 to 11, for providing long-lasting hold, high-setting properties, and elasticity to the hair style.

15. A method for preparing a cosmetic composition as defined in claims 1 to 12, said method comprising the steps of:
(a) providing a polymer comprising from 1% to 100% of polymerized units (A);
(b) forming direct covalent bonds between the alpha carbons of two different units (A) of said polymers by break of the alpha-hydrogen-bond relative to R₃, said bonds comprising no intermediate molecule, in order to obtain elastomeric polymers;
(c) providing a cosmetic composition comprising a cosmetically acceptable carrier and optionally at least one cosmetic active ingredient;
(d) incorporating said elastomeric polymers into said cosmetic composition.

16. A method for preparing a cosmetic composition as defined in claims 1 to 12, said method comprising the steps of:
(a) providing a cosmetic composition comprising a cosmetically acceptable carrier, and optionally at least one cosmetic active ingredient;
(b) incorporating a polymer comprising from 1% to 100% of polymerized units (A) into said cosmetic composition.
(c) forming direct covalent bonds between the alpha carbons of two different units (A) of said polymers by break of the alpha-hydrogen-bond relative to R₃, said bonds comprising no intermediate molecule, in order to obtain elastomeric polymers;

17. A method for applying a cosmetic composition onto hair and/or skin, said method comprising the steps of:
(a) providing a cosmetic composition as defined in claims 1 to 12 comprising, in a cosmetically acceptable carrier, elastomeric polymers comprising from 1% to 100% of polymerized units (A) wherein direct covalent bonds are formed between the alpha carbons of two different units (A) by break of the alpha-hydrogen-bond relative to R₃ and wherein said bonds comprise no intermediary molecule, and optionally at least one cosmetic active ingredient;
(b) applying said composition onto skin and/or hair
